# EUROPEAN PATENT APPLICATION

(11) **EP 4 700 783 A1**
(43) Date of publication of application: **25.02.2026**
(21) Application number: 24792991.2
(22) Date of filing: 17.04.2024
(51) Int. Cl.: G16H 15/00, G16B 50/00, G16B 40/10, G16B 20/20, G16B 40/20, G16H 10/60, G16H 70/00, G16H 10/40, G16H 40/20, G16B 35/00

(54) **METHOD FOR AUTOMATICALLY GENERATING PROPOSAL FOR DEVELOPMENT FOR IN VITRO DIAGNOSTIC KIT, AND SERVER THEREFOR**

(30) Priority: 17.04.2023 KR 20230049783
(71) Applicant: Seegene, Inc., Seoul 05548 (KR)
(72) Inventor: CHUN, Jong Yoon, Yeoju-si Gyeonggi-do 12601 (KR)
(74) Representative: Viering, Jentschura & Partner mbB Patent- und Rechtsanwälte
(86) International application number: PCT/KR2024/005127
(87) International publication number: WO 2024/219806

(57) **Abstract**

A method for automatically generating a research and development proposal for an in vitro diagnostic kit, performed by a server for automatically generating the research and development proposal, includes: acquiring an initial proposal including intended use information for the kit; providing the acquired initial proposal to a pre-trained language model; acquiring a specification (spec) for the kit from the language model receiving the initial proposal, wherein the acquired specification for the kit includes at least one of a technical specification to be used for the research and development of the kit, a resource specification for the research and development, a design specification for the kit, a regulatory approval specification that is needed to meet for approval, and a performance specification for the kit; and controlling to generate the research and development proposal for the kit including the acquired specification and the intended use information for the kit.

## Description

### TECHNICAL FIELD

The present invention relates to a method for automatically generating a research and development proposal for an in-vitro diagnostic kit and a server therefor.

### BACKGROUND ART

In-vitro diagnostic kits may be researched and developed in various ways. For example, the sales or product planning department of a company that develops kits collects and confirms development requests for kits capable of diagnosing specific diseases or conditions from medical professionals such as laboratory physicians. Such a development request may be referred to as intended use information. Furthermore, the aforementioned company reviews such a development request from various aspects. For example, marketability, as well as the cost or time for development, may be reviewed. In addition, the company reviews various information, including papers and patents related to such diseases or conditions. In particular, since biological papers may directly or indirectly mention information necessary for the development of a kit, a review of these papers is performed. Once the review is completed, a research and development proposal (proposal) for the kit is generated based on it. Then, based on the generated research and development proposal, it is determined whether to proceed with research and development for the kit.

However, the number of biological papers published each year is not small. According to one study, the number of articles on MDx markers published in 2022 exceeded 30,000. Therefore, it is practically impossible for the aforementioned company to review these published papers one by one in detail.

Accordingly, after papers are selected using keywords, a review is often conducted centering on these selected papers. However, in the internet age, technology is being updated every day, and papers are also being published in real time. Therefore, even if papers are selected using keywords, the number of selected papers is not small. Furthermore, since other papers may be published during the time from searching for papers to starting development, it is not practically easy to reflect the newest technology in the development of the kit.

### DETAILED DESCRIPTION OF THE INVENTION

### TECHNICAL PROBLEMS

A problem to be solved according to an embodiment includes providing a technology for automatically generating a research and development proposal for various types of diagnostic kits, for example, in-vitro diagnostic kits.

For example, the problem may include automatically generating a research and development proposal for a kit by using intended use information for the kit that is automatically collected from medical professionals and various types of documents or information, such as biological papers or patents, which are automatically searched.

However, the problem to be solved according to an embodiment is not limited thereto.

### MEANS FOR SOLVING PROBLEMS

A method for automatically generating a research and development proposal for an in vitro diagnostic kit, performed by a server for automatically generating the research and development proposal according to a first embodiment, includes: the steps of acquiring an initial proposal including intended use information for the kit; providing the acquired initial proposal to a pre-trained language model; acquiring a specification (spec) for the kit from the language model receiving the initial proposal, wherein the acquired specification for the kit includes at least one of a technical specification to be used for the research and development of the kit, a resource specification for the research and development, a design specification for the kit, a regulatory approval specification that is needed to meet for approval, and a performance specification for the kit; and controlling to generate the research and development proposal for the kit including the acquired specification and the intended use information for the kit.

Further, the intended use information may include at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

Further, the initial proposal may be created and acquired as a form prepared to allow inputting predetermined items is provided to a first mover who has proposed the intended use information, and the intended use information may include at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

Further, the predetermined items may further include at least one of identity information of the first mover, information on whether the first mover possesses clinical samples, an amount of the possessed clinical samples, and information on a stage of the research and development of the kit in which the first mover wishes to participate, and the method may further include, if at least one of the identity information of the first mover, the information on whether the first mover possesses clinical samples, and the intended use information as an essential information, among the predetermined items is not input in the initial proposal, requesting the first mover to provide a description for the missing essential information.

Further, the language model may be a generative model trained by RLHF (Reinforcement Learning from Human Feedback).

Further, the RLHF may include performing a learning process using a dialogue set generated by a human and a learning process using a ranking selected by a human for a plurality of outputs generated by the language model.

Further, at least one of a patent, a paper, and a research note may be used for training the language model, and if the research note is used for the training, the research note may include experiment result information reported in a development completion review of the kit.

Further, the language model may extract, from a plurality of papers, at least one piece of paper information among authors of the plurality of papers and institutions to which the authors of the papers belong, and the research and development proposal may be generated to include the at least one extracted piece of paper information.

Further, the technical specification of the kit may include at least one of a sampling technology, an extraction technology, a test reaction setup technology, a target signal generation technology, a signal analysis technology, and an information display technology.

Further, the kit may be a molecular diagnostic kit, and the technical specification of the molecular diagnostic kit may include at least one of oligo type (primer only or primer and probe), oligo structure, target-signal generation mechanism with amplification, amplification curve analysis, amplification curve viewing, and liquid handler operation information.

Further, the resource specification for the research and development of the kit may include at least one of information on availability of clinical samples, an amount of the possessed clinical samples if possessed, a source of the clinical samples, personnel, research funds, a research period, research equipment, and research facilities.

Further, the design specification of the kit may include information on at least one of an analyte panel configuration, a channel configuration, an IC, a PC, and an NC.

Further, the regulatory approval specification may include at least one of numerical data that is needed to satisfy to obtain approval, target analyte information for an approval process, panel configuration information according to a type of approval, information on a source of clinical samples, a target country for the approval, and information on an institution where experiments for the approval are conducted.

Further, the performance specification of the kit may include at least one of sensitivity, specificity, TAT (Turnaround Time), reproducibility indicating consistency of diagnostic results when a same sample is tested at different times or in different places, linearity indicating an accuracy of test results as a sample concentration increases, a limit of detection (LoD) indicating a minimum detectable sample concentration, and a range of inclusive and exclusive strains.

Further, research and development documents of a kit of a same or different type as the kit may be used in a training process of the language model.

Further, the method may further include: acquiring marketability survey data for the kit from the language model that received the initial proposal, wherein the research and development proposal for the kit may be generated to further include the acquired marketability survey data.

Further, the method may further include: acquiring information necessary for production of the kit from the language model that received the initial proposal, wherein the research and development proposal for the kit may be generated to further include the acquired information necessary for the production of the kit.

Further, the method may further include: acquiring at least one of information regarding a target price (TP) for sale of the kit and projected sales revenue from the language model that received the initial proposal, wherein the research and development proposal for the kit may be generated to further include at least one of the acquired information regarding the target price and the information regarding the projected sales revenue.

Further, one or more required specification items that is needed to be included in the research and development proposal may be predefined in the server for automatically generating the research and development proposal, and in the generating the research and development proposal for the kit, if at least a part of the one or more required specification items is missing from the acquired kit specification, the missing required specification item may be indicated in the generated research and development proposal, or the research and development proposal is not generated.

Further, the method may further include: when the language model acquires supplementary information for the missing required specification item from an updated or new paper and/or patent, transmitting an alarm to a first mover who proposed the intended use information and/or modifying or generating the research and development proposal to include the supplementary information for the missing required specification item.

Further, in the research and development proposal, an author of the research and development proposal may be described as at least one of a provider who provided the intended use information and an operating entity of the server for automatically generating the research and development proposal.

Further, the method may further include: selecting an evaluator to evaluate the research and development proposal.

Further, the evaluator may be selected as at least one of an author of the paper, an operating entity of a publication server where the paper is published, a person selected by the operating entity, the server for automatically generating the research and development proposal, and a person selected by the operating entity of the server for automatically generating the research and development proposal.

Further, the method may further include: acquiring an evaluation result for the research and development proposal; and summarizing and providing the acquired evaluation result.

Further, the in vitro diagnostic kit may include at least one of a molecular diagnostic kit, an immunodiagnostic kit, and an antigen/antibody diagnostic kit.

Further, the method may further include: controlling performance of a review on at least one of the acquired initial proposal and the specification acquired for the kit.

Further, the method may further include: evaluating an expertise of a first mover who has proposed the initial proposal by using a background knowledge of the first mover, wherein whether to perform the review may be determined based on a result of the evaluation of the background knowledge of the first mover.

Further, the method may further include: controlling performance of a review on the generated research and development proposal.

A computer-readable recording medium according to a second embodiment may store a computer program programmed to perform each step included in the method described above.

A computer program according to a third embodiment may be stored on a computer-readable recording medium, and the computer program may be programmed to perform each step included in the method described above.

A server for automatically generating a research and development proposal according to a fourth embodiment, includes: a memory storing at least one instruction; and a processor, wherein when the at least one instruction is executed by the processor, an initial proposal including intended use information for an in vitro diagnostic kit is acquired, the acquired initial proposal is provided to a pre-trained language model, a specification (spec) for the kit is acquired from the language model that received the initial proposal, wherein the acquired specification for the kit includes at least one of a technical specification to be used for the research and development of the kit, a resource specification for the research and development, a design specification for the kit, a regulatory approval specification that is needed to meet for approval, and a performance specification for the kit, and a research and development proposal for the kit is generated to include the acquired specification for the kit and the intended use information.

Further, the intended use information may include at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

Further, the initial proposal may be created and acquired as a form prepared to include predetermined items is provided to a first mover who proposed the intended use information, and the intended use information may include at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

Further, the predetermined items may further include at least one of identity information of the first mover, whether clinical samples are possessed, an amount of the possessed clinical samples, and information on a stage of the research and development of the kit in which the first mover wishes to participate, and when the at least one instruction is executed by the processor, if at least one of the identity information of the first mover, the information on whether clinical samples are possessed, and the intended use information, which correspond to required information among the predetermined items, is not included in the initial proposal, the first mover may be requested to provide a description for the corresponding item.

Further, the language model may be a generative model trained by RLHF (Reinforcement Learning from Human Feedback).

Further, the RLHF may include performing a learning process using a dialogue set generated by a human and a learning process using a ranking selected by a human for a plurality of outputs generated by the language model.

Further, at least one of a patent, a paper, and a research note may be used for training the language model, and if the research note is used for the training, the research note may include experiment result information reported in a development completion review of the kit.

Further, the language model may extract, from a plurality of papers, at least one piece of paper information among authors of the plurality of papers and institutions to which the authors of the papers belong, and the research and development proposal may be generated to include the at least one extracted piece of paper information.

Further, the technical specification of the kit may include at least one of a sampling technology, an extraction technology, a test reaction setup technology, a target signal generation technology, a signal analysis technology, and an information display technology.

Further, the kit may be a molecular diagnostic kit, and the technical specification of the molecular diagnostic kit may include at least one of oligo type (primer only or primer and probe), oligo structure, target-signal generation mechanism with amplification, amplification curve analysis, amplification curve viewing, and liquid handler operation information.

Further, the resource specification for the research and development of the kit may include at least one of information on availability of clinical samples, an amount of the possessed clinical samples if possessed, a source of the clinical samples, personnel, research funds, a research period, research equipment, and research facilities.

Further, the design specification of the kit may include information on at least one of an analyte panel configuration, a channel configuration, an IC, a PC, and an NC.

Further, the regulatory approval specification may include at least one of numerical data that the kit must satisfy to obtain approval, target analyte information for an approval process, panel configuration information according to a type of approval, information on a source of clinical samples, a target country for the approval, and information on an institution where experiments for the approval are conducted.

Further, the performance specification of the kit may include at least one of sensitivity, specificity, TAT (Turnaround Time), reproducibility indicating consistency of diagnostic results when a same sample is tested at different times or in different places, linearity indicating an accuracy of test results as a sample concentration increases, a limit of detection (LoD, Limit of Detection) indicating a minimum detectable sample concentration, and a range of inclusive and exclusive strains.

Further, research and development documents of a kit of a same or different type as the kit may be used in a training process of the language model.

Further, when the at least one instruction is executed by the processor, marketability survey data for the kit may be acquired from the language model that received the initial proposal, and the research and development proposal for the kit may be generated to further include the acquired marketability survey data.

Further, when the at least one instruction is executed by the processor, information necessary for production of the kit may be acquired from the language model that received the initial proposal, and the research and development proposal for the kit may be generated to further include the acquired information necessary for the production of the kit.

Further, when the at least one instruction is executed by the processor, at least one of information regarding a target price for sale of the kit and projected sales revenue may be acquired from the language model that received the initial proposal, and the research and development proposal for the kit may be generated to further include at least one of the acquired information regarding the target price and the information regarding the projected sales revenue.

Further, one or more required specification items that is needed to be included in the research and development proposal may be predefined and stored in the memory, and the research and development proposal may be generated with the missing required specification item indicated, or may not be generated, when at least a part of the one or more required specification items is missing from the acquired kit specification.

Further, if the language model acquires supplementary information for the missing required specification item from an updated or new paper and/or patent, an alarm may be transmitted to a first mover who proposed the intended use information and/or the research and development proposal may be modified or generated to include the supplementary information for the missing required specification item.

Further, in the research and development proposal, an author of the research and development proposal may be described as at least one of a provider who provided the intended use information and an operating entity of the server for automatically generating the research and development proposal.

Further, when the at least one instruction is executed by the processor, an evaluator to evaluate the research and development proposal may be selected.

Further, the evaluator may be selected as at least one of an author of the paper, an operating entity of a publication server where the paper is published, a person selected by the operating entity, an operating entity of the server for automatically generating the research and development proposal, and a person selected by the operating entity of the server for automatically generating the research and development proposal.

Further, when the at least one instruction is executed by the processor, an evaluation result for the research and development proposal may be acquired, and the acquired evaluation result may be summarized and provided.

Further, the in vitro diagnostic kit may include at least one of a molecular diagnostic kit, an immunodiagnostic kit, and an antigen/antibody diagnostic kit.

Further, when the at least one instruction is executed by the processor, a review may be controlled to be performed on at least one of the acquired initial proposal and the specification acquired for the kit.

Further, when the at least one instruction is executed by the processor, an expertise of a first mover who proposed the initial proposal may be evaluated by using a background knowledge of the first mover, and whether to perform the review may be determined based on a result of the evaluation of the background knowledge of the first mover.

Further, when the at least one instruction is executed by the processor, a review of the generated research and development proposal may be controlled to be performed.

### EFFECTS OF THE INVENTION

According to an embodiment, a final proposal, which is a research and development proposal for a kit, may be generated from various types of documents using a first mover's initial proposal. Furthermore, information regarding marketability, regulatory approval, and production may also be reflected in this final proposal. Therefore, from the perspective of a company that researches and develops kits, it is possible to acquire necessary up-to-date information from vast amounts of information in the shortest time without errors, and from that, even secure a research and development proposal, without a person needing to examine documents such as papers or patent literature to review the first mover's initial proposal. Through this, the feasibility of research and development for various types of kits may be reviewed quickly and accurately.

### BRIEF DESCRIPTION OF THE DRAWINGS

FIG. 1 exemplarily illustrates a concept of automatically generating a research and development proposal according to an embodiment.
FIG. 2 exemplarily illustrates a configuration in which a server for automatically generating the research and development proposal is connected on a network according to the embodiment.
FIG. 3 conceptually illustrates a block diagram of the server for automatically generating the research and development proposal according to the embodiment.
FIG. 4 conceptually illustrates an architecture for deep learning.
FIG. 5 and FIG. 6 exemplarily illustrate a process performed in pre-training during transfer learning.
FIG. 7 exemplarily illustrates a flowchart showing a procedure for training a language model according to the embodiment.
FIG. 8 exemplarily illustrates a flowchart of a method for automatically generating the research and development proposal according to the embodiment.

### MODE FOR IMPLEMENTING THE INVENTION

Advantages and features of the present invention, and implementation methods thereof will be clarified through following embodiments described with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present disclosure to those skilled in the art. Further, the present invention is only defined by scopes of claims.

In describing the embodiments of the present invention, the detailed descriptions of well-known functions or configurations will be omitted if it is determined that the detailed descriptions of well-known functions or configurations may unnecessarily make obscure the spirit of the present invention. The terms used henceforth are defined in consideration of the functions of the present invention and may be altered according to the intent of a user or operator, or conventional practice. Therefore, the terms should be defined on the basis of the entire content of this disclosure.

First, before describing the present invention, terms will be examined.

An in-vitro diagnostic kit (kit) may be for diagnosis, but is not limited thereto. For example, the kit may be for diagnosing a pathogen, a pathogenic gene, or an SNP, or for diagnosing a disease name or an infectious disease name.

Such in-vitro diagnostic kits may include a molecular diagnostic kit, an immunodiagnostic kit, and/or an antigen/antibody diagnostic kit, and the molecular diagnostic kit may include a NAAT (Nucleic Acid Amplification Test) kit, but is not limited thereto.

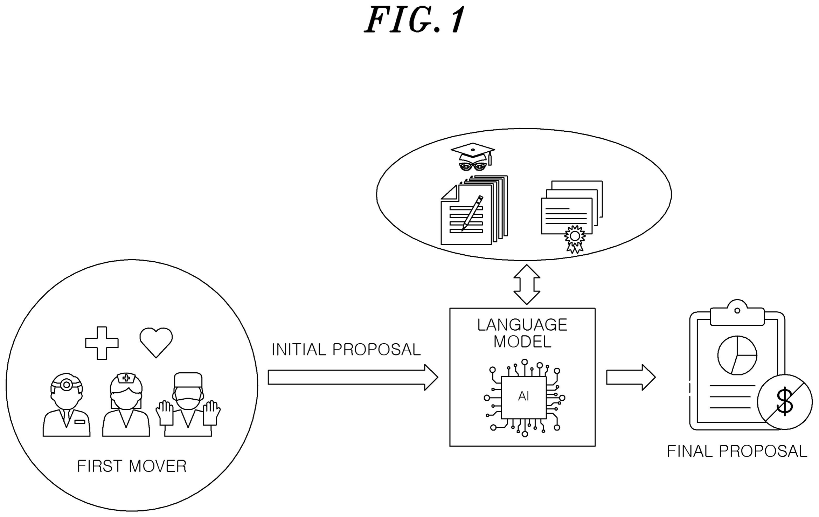

FIG. 1 exemplarily illustrates a concept of automatically generating a research and development proposal for an in-vitro diagnostic kit according to an embodiment. However, FIG. 1 is merely exemplary, and the spirit of the present invention is not to be interpreted as being limited to what is illustrated in FIG. 1.

Referring to FIG. 1, a first mover is illustrated. The first mover may be a medical professional as illustrated in FIG. 1, but is not limited thereto. Specifically, anyone who may recognize the need for a diagnosis regarding the cause or stage of a certain disease, condition, or symptom may be a first mover.

The first mover provides an initial proposal to a language model. The initial proposal may be a type of information or a draft proposal. Such an initial proposal may include a request or query that 'a diagnosis is needed for the cause of a certain disease, condition, or symptom', which may be more specifically referred to as intended use information. If such a language model is implemented as a generative language model, the aforementioned initial proposal may be provided to this generative language model through the RAG (Retrieval-Augmented Generation) technique, but is not limited thereto.

Meanwhile, the aforementioned intended use information may include at least one of the following items, but is not limited thereto.

- Diagnostic test purpose
- Diagnostic test method
- Specimen type
- Target analyte information to be detected or analyzed
- Pathogen information
- Information on contaminants
- Information about a pathogenic gene or SNP (Single Nucleotide Polymorphism)
- Disease name
- Infectious disease name
- Symptom information

The intended use information will be described in more detail. Medical professionals and others want to make an accurate diagnosis of a person visiting a medical institution. This is because only then is an accurate prescription possible. Accordingly, a medical professional wants to know things like 'if there is a certain symptom, what cause or disease it is due to, what pathogen is causing it, what the target analyte is,' and so on. What is desired to be known in this way may be an example of intended use information, but is not limited thereto.

Meanwhile, in addition to the aforementioned intended use information, the initial proposal may include at least one of the items described below, but is not limited thereto.
- Identity information of the first mover
- Whether clinical samples are possessed
- Amount of possessed clinical samples
- Information regarding the stage of the kit's R&D in which the first mover wishes to participate

The aforementioned initial proposal is incomplete. Specifically, a company seeking to research and develop a kit cannot develop the kit based only on the content described in such an initial proposal. To embark on R&D, various papers, patents, research notes, or regulatory approval information of previously approved in-vitro diagnostic kits must be referenced. Furthermore, marketability must be reviewed, and the feasibility of actual implementation must also be reviewed. In addition, various other matters must be comprehensively reviewed.

That is, the aforementioned company reviews a final proposal in which several items are additionally described in addition to the content described in the initial proposal, and then, if it is feasible, embarks on R&D.

At this time, the items to be described in this final proposal may be at least one of the following, but are not limited thereto.
- Identity information of the first mover
- Identity information of the author of the final proposal
- Specification (spec) of the kit
- Intended use information
- Marketability information
- Information necessary for regulatory approval
- Information necessary for production
- Information regarding Target Price (TP) and projected sales revenue

Among these, the 'kit's spec' may include at least one of a technical specification to be used for the R&D of the kit, a resource specification for the R&D, a design specification for the kit, a regulatory approval specification that the kit must meet to obtain approval, and a performance specification of the kit.

To examine this in detail, the technical specification may include at least one of the following.
- Sampling technology
- Extraction technology
- Reaction setup
- Target signal generation technology
- Signal analysis
- Information display technology

Alternatively, the aforementioned technical specification may include at least one of the following.
- Oligo Type (primer only or primer and probe) or Structure
- Target-Signal Generation Mechanism with Amplification
- Amplification Curve Analysis
- Amplification Curve Viewing
- Liquid Handler Operation

Next, the resource specification may include at least one of: information on the availability of clinical samples, the amount of available clinical samples if any, the source of the clinical samples, personnel, research funds, research period, research equipment, and research facilities.

Next, the design specification may include at least one of the following.
- Analyte panel configuration
- Channel configuration
- IC (internal control)
- PC (positive control)
- NC (negative control)

Next, the regulatory approval specification may include at least one of: numerical data that the kit must satisfy to obtain approval, target analyte information for the approval process, panel configuration information according to the type of approval, information on the source of clinical samples, the target country for the approval, and the institution where experiments for the approval are conducted.

Next, the performance specification may include at least one of the following.
- Sensitivity
- Specificity
- TAT (Turnaround Time)
- Reproducibility indicating the consistency of diagnostic results when the same sample is tested at different times or places
- Linearity indicating the accuracy of test results as sample concentration increases
- LoD (Limit of Detection), which indicates the minimum detectable sample concentration
- Range of inclusive and exclusive strains

Here, it is a matter of course that what is included in a specific specification may also be included in another specification.

Meanwhile, the items included in the aforementioned specifications may be interpreted as being related to in-vitro diagnostics, particularly molecular diagnostics. For example, 'sampling' may refer to an act of collecting a sample, and 'extraction' may refer to an act of extracting a nucleic acid or the like from the collected sample. 'Amplification' may refer to an act of amplifying a nucleic acid, and 'information display' may refer to an act of displaying information that is the result of nucleic acid amplification. Of course, it is also possible that the items included in the aforementioned specifications may be interpreted as being related to other diagnostics, such as immunodiagnostics, rather than molecular diagnostics.

The final proposal is generated using a language model as illustrated in FIG. 1. Specifically, referring to FIG. 1, the language model is provided with an initial proposal including intended use information from a first mover. Then, the language model acquires the content to be described in the final proposal from numerous papers or patents based on the content described in the initial proposal. For this purpose, a technology based on RAG (Retrieval-Augmented Generation) may be employed in this language model. Then, a final proposal is generated using the acquired content.

At this time, the language model may pre-train in advance on various types of papers, patent literature, publicly available research notes, or information on conditions for regulatory approval, or it may access these papers or patent literature based on already learned knowledge and acquire desired information therefrom. Furthermore, although not illustrated in FIG. 1, in addition to such papers and patent literature, for various contents that should be described in the final proposal, such as marketability information or regulatory approval information, the language model may acquire them using documents extensively uploaded on the internet and then provide them.

That is, according to the embodiment, a final proposal, which is a research and development proposal for a kit, may be generated from numerous papers and patent literature using the first mover's initial proposal with a language model, and additionally, information on marketability, regulatory approval, production, and the like may also be acquired from this language model and reflected in the final proposal. Therefore, from the perspective of a company that researches and develops kits, it is possible to acquire necessary up-to-date information from vast amounts of information in the shortest time without errors, and from that, even secure a research and development proposal, without a researcher needing to examine papers or patent literature one by one to review the first mover's request. Through this, the feasibility of research and development for various types of kits may be reviewed quickly and accurately.

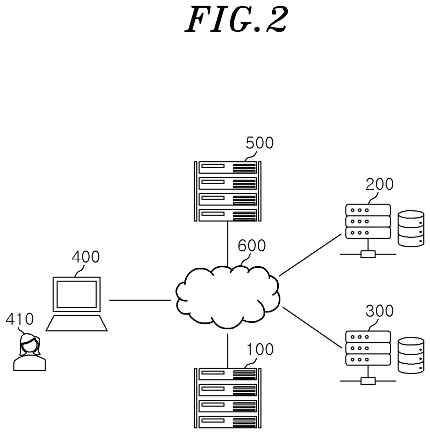

FIG. 2 exemplarily illustrates a configuration in which a server for generating a research and development proposal is connected on a network according to the embodiment.

Referring to FIG. 2, the server for automatically generating a research and development proposal 100 according to the embodiment may be connected to a user terminal 400 and the like via a network 600. Here, FIG. 2 is merely exemplary, and the spirit of the present invention is not to be interpreted as being limited to what is illustrated in FIG. 2.

Here, the network 600 means a wireless or wired network. Among these, a wireless network may include at least one of LTE (long-term evolution), LTE-A (LTE Advance), CDMA (code division multiple access), WCDMA (wideband CDMA), UMTS (universal mobile telecommunications system), WiBro (Wireless Broadband), WiFi (wireless fidelity), Bluetooth, NFC (near field communication), and GNSS (global navigation satellite system). Furthermore, a wired network may include at least one of USB (universal serial bus), HDMI (high definition multimedia interface), RS-232 (recommended standard 232), LAN (Local Area Network), WAN (Wide Area Network), the internet, and a telephone network.

Next, the user terminal 400 is the terminal of the first mover. Such a user terminal 400 may include a smartphone, tablet PC, desktop PC, or server, as illustrated in FIG. 2. The first mover may provide an initial proposal including the aforementioned intended use information to the server 100 through such a user terminal 400.

A paper publication server 200 is a server where various papers are published. For example, a server operated by an institution like Nature may be the paper publication server 200. On such a paper publication server 200, from thousands to tens or hundreds of thousands of papers are published annually. It is a matter of course that biological papers are included among these published papers.

A patent document publication server 300 is a server where various patent documents are published. Such a patent document publication server 300 may exist in each country.

Meanwhile, although not illustrated in FIG. 2, a language model is provided. This language model may be provided in the server 100, or it may be provided in a separate server that generates and operates the language model, which is not illustrated in FIG. 2. If it is the latter, the server 100 may access the language model provided in such a separate server and request it to perform a desired function, for example, generating a final proposal for a research and development proposal. Hereinafter, the description will be based on the premise that this language model is not provided in the server 100 but in a separate server, but the present invention is not limited to this interpretation, and an embodiment in which it is provided in the server 100 is not excluded from the present invention.

A R&D proposal generation server 100 is implemented to generate a research and development proposal for a kit, i.e., a final proposal. Such an R&D proposal generation server 100 may be implemented in a server or the like, but is not limited thereto. Hereinafter, the R&D proposal generation server 100 will be examined in more detail.

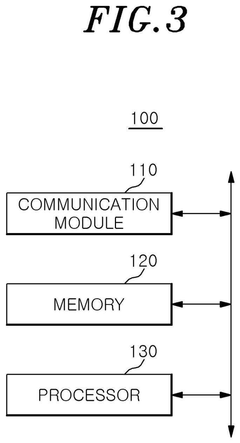

FIG. 3 is an exemplary configuration diagram of a server for automatically generating the R&D proposal 100 according to the embodiment. Referring to FIG. 3, the R&D proposal generation server 100 includes a communication module 110, a memory 120, and a processor 130. However, the configuration diagram illustrated in FIG. 3 is merely exemplary, and the spirit of the present invention is not to be interpreted as being limited by the configuration diagram illustrated in FIG. 3. For example, the R&D proposal generation server 100 may include at least one component not illustrated in FIG. 3 or may not include at least one of the components illustrated in FIG. 3.

The communication module 110 may be implemented by a wired or wireless communication module. The R&D proposal generation server 100 may communicate with external terminals, for example, the various types of terminals or servers 200, 300, and 400 illustrated in FIG. 2, through this communication module 110.

The memory 120 may be implemented by a medium that stores information. Such a medium may be a storage medium of at least one type among flash memory type, hard disk type, multimedia card micro type, card type memory (e.g., SD or XD memory, etc.), Random Access Memory (RAM), Static Random Access Memory (SRAM), Read-Only Memory (ROM), Electrically Erasable Programmable Read-Only Memory (EEPROM), Programmable Read-Only Memory (PROM), magnetic memory, magnetic disk, and optical disk, but is not limited thereto.

Various types of information may be stored in the memory 120. For example, information acquired by the R&D proposal generation server 100 from external terminals or servers 200, 300, and 400 through the communication module 110 may be stored in the memory 120. Furthermore, a plurality of training data to be used for training various types of models or modules to be described later may be stored in the memory 120.

Furthermore, various types of modules or models may be implemented in the memory 120. If such a module or model is executed by the processor 130, which will be described later, a desired function is performed. Each module or model will be described later.

Next, the processor 130 will be described. First, the processor 130 according to the embodiment may perform the technical features according to the embodiments of the present invention to be described later by executing at least one instruction stored in the memory 120. In the embodiment, the processor 130 may be composed of at least one core and may include a processor for data analysis and/or processing, such as a central processing unit (CPU) of the R&D proposal generation server 100, a general-purpose graphics processing unit (GPGPU), or a tensor processing unit (TPU).

The processor 130 may train a neural network or model designed with a machine learning or deep learning method. To this end, the processor 130 may perform calculations for training the neural network, such as processing input data for training, extracting features from the input data, calculating errors, and updating the weights of the neural network using backpropagation.

Furthermore, the processor 130 may perform inference for a predetermined purpose using a model implemented as an artificial neural network.

Hereinafter, an artificial neural network will be examined. A model in this specification may mean any form of computer program that operates based on a network function, an artificial neural network, and/or a neural network. Throughout this specification, model, neural network, network function, and neural network may be used interchangeably. A neural network is formed by one or more nodes interconnected through one or more links, forming an input node and output node relationship within the neural network. The characteristics of a neural network may be determined by the number of nodes and links within the neural network, the association between the nodes and links, and the value of the weight assigned to each of the links. A neural network may be composed of a set of one or more nodes. A subset of the nodes constituting the neural network may form a layer.

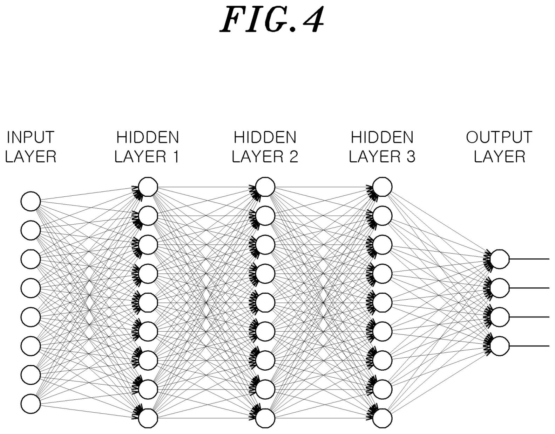

A deep neural network (DNN) may mean a neural network that includes a plurality of hidden layers in addition to an input layer and an output layer, and as conceptually illustrated in FIG. 4, the hidden layers in the middle are composed of one or more, preferably two or more, layers in a deep neural network.

Such a deep neural network may include a convolutional neural network (CNN), a vision transformer, a recurrent neural network (RNN), a Long Short Term Memory (LSTM) network, a Generative Pre-trained Transformer (GPT), an auto encoder, Generative Adversarial Networks (GAN), a restricted Boltzmann machine (RBM), a deep belief network (DBN), a Q network, a U network, a Siamese network, a Generative Adversarial Network (GAN), a transformer, and the like.

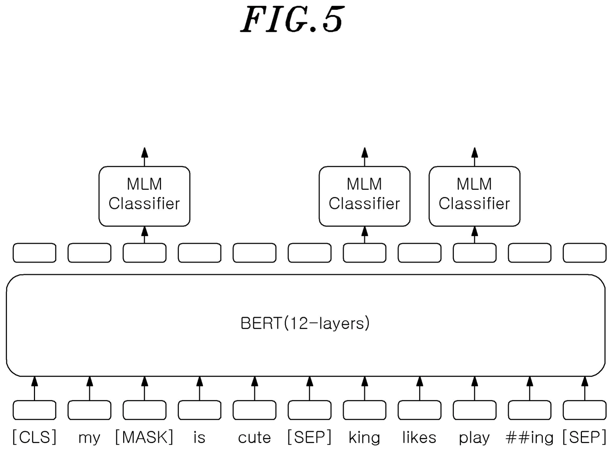

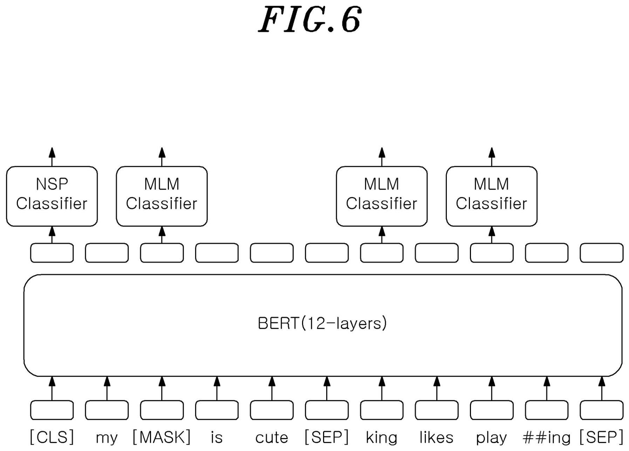

Alternatively, according to the embodiment, the deep neural network may be a model trained by a transfer learning method. Here, the transfer learning method is a learning method that obtains a pre-trained model (or base part) having a first task by pre-training a large amount of unlabeled training data using a semi-supervised or self-supervised learning method through techniques illustrated in FIG. 5 and FIG. 6 (MLM and NSP), and then implements a target model by training labeled training data using a supervised learning method to fine-tune the pre-trained model to be suitable for a second task. One of the models trained by the transfer learning method includes, but is not limited to, BERT (Bidirectional Encoder Representations from Transformers) or GPT.

The description of the aforementioned deep neural network is merely an example, and the present disclosure is not limited thereto. Here, the aforementioned convolutional neural network is composed of a feature learning part that extracts features from an image, and a classification part that performs classification using the extracted features. The feature learning part may include a convolutional layer where features are extracted from an image using a kernel, a ReLU layer which is one of the activation functions, and a pooling layer for reducing the dimension of the data, but is not limited thereto. Furthermore, the classification part may include a flatten layer that arranges the features extracted from the feature learning part in a line, and a fully connected layer and a softmax function where classification is substantially performed, but is not limited thereto.

The neural network may be trained by at least one method among supervised learning, unsupervised learning, semi-supervised learning, self-supervised learning, or reinforcement learning. Training a neural network may be a process of applying, to the neural network, knowledge for the neural network to perform a specific operation.

A neural network may be trained in a direction that minimizes output errors. Training a neural network is a process that includes iteratively inputting training data into the neural network, calculating an error between an output of the neural network for the training data and a target, and backpropagating the error from an output layer to an input layer of the neural network in a direction to reduce the error to update a weight of each node of the neural network. In the case of supervised learning, labeled data in which a correct answer is labeled for each training data is used, and in the case of unsupervised learning, unlabeled data in which a correct answer is not labeled for each training data may be used. The amount of change in the connection weight of each updated node may be determined according to a learning rate. The calculation of the neural network for the input data and the backpropagation of the error may constitute an epoch. The learning rate may be applied differently according to the number of repetitions of the training cycle of the neural network. Furthermore, to prevent overfitting, methods such as increasing training data, regularization, dropout which deactivates some nodes, and a batch normalization layer may be applied.

Meanwhile, the model disclosed in the embodiment may borrow at least a part of a transformer. A transformer may be composed of an encoder that encodes embedded data and a decoder that decodes the encoded data. A transformer may have a structure that receives a series of data and outputs a series of data of a different type through encoding and decoding steps. In an embodiment, the series of data may be processed into a form that a transformer may compute. The process of processing the series of data into a form that a transformer may compute may include an embedding process. Expressions such as data token, embedding vector, embedding token, etc., may refer to data embedded in a form that a transformer may process.

For the transformer to encode and decode a series of data, the encoders and decoders within the transformer may process them using an attention algorithm. The attention algorithm may mean an algorithm that, for a given Query, finds the similarity to one or more Keys, reflects this given similarity in the Value corresponding to each Key, and then calculates an attention value by taking a weighted sum of the Values with the reflected similarity.

Depending on how the query, key, and value are set, various types of attention algorithms may be classified. For example, if attention is obtained by setting the query, key, and value all the same, this may mean a self-attention algorithm. To process an input series of data in parallel, if attention is obtained by reducing the dimension of the embedding vector and obtaining a separate attention head for each divided embedding vector, this may mean a multi-head attention algorithm.

In the embodiment, the transformer may be composed of modules that perform a plurality of multi-head self-attention algorithms or multi-head encoder-decoder algorithms. In the embodiment, the transformer may also include additional components other than the attention algorithm, such as embedding, normalization, softmax, etc. The method of constructing a transformer using an attention algorithm may include the method disclosed in Vaswani et al., Attention Is All You Need, 2017 NIPS, which is incorporated herein by reference.

The transformer may be applied to various data domains, such as embedded natural language, segmented image data, or audio waveforms, to transform a series of input data into a series of output data. To convert data having various data domains into a series of data that may be input to the transformer, the transformer may embed the data. The transformer may process additional data representing the relative positional relationship or phase relationship between a series of input data. Alternatively, a series of input data may be embedded by additionally reflecting vectors representing the relative positional relationship or phase relationship between the input data in the series of input data. As an example, the relative positional relationship between a series of input data may include, but is not limited to, the word order within a natural language sentence, the relative positional relationship of each segmented image, the time order of a segmented audio waveform, and the like. The process of adding information representing the relative positional relationship or phase relationship between a series of input data may be referred to as positional encoding.

Hereinafter, various operations or functions that the R&D proposal generation server 100 may perform by executing at least one instruction stored in the memory 120 by the processor 130 will be described.

First, the processor 130 may control the communication module 110. Through this, the R&D proposal generation server 100 may acquire information by communicating with various terminals or servers 200, 300, and 400 illustrated in FIG. 2 through the communication module 110.

Furthermore, the processor 130 may read the aforementioned data or instructions stored in the memory 120, and may write new data or instructions to the memory 120. Furthermore, the processor 130 may modify or delete already recorded data or instructions.

Furthermore, the processor 130 may execute various models or modules stored in the memory 120. Here, such models or modules may be implemented by the aforementioned artificial neural network method or a rule-based method.

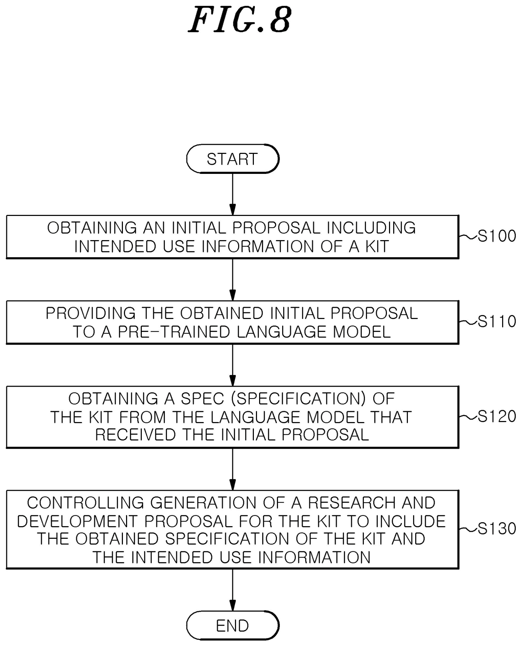

Now, the operation of the R&D proposal generation server 100 will be described with reference to FIG. 8.

First, FIG. 8 exemplarily illustrates a flowchart of a method for generating the R&D proposal according to the embodiment if this method is performed by the R&D proposal generation server 100. Here, the flowchart is merely exemplary, and the spirit of the present invention is not limited thereto. For example, according to the embodiment, each step may be performed in a different order from that illustrated in FIG. 8, or at least one step not illustrated in FIG. 8 may be additionally performed, or at least one of the steps illustrated in FIG. 8 may not be performed.

Referring to FIG. 8, a step S100 of acquiring an initial proposal including intended use information for an in-vitro diagnostic kit is performed. At this time, the type of kit is not to be interpreted as being limited to in-vitro diagnostic use.

Furthermore, a step S110 of providing the acquired initial proposal to a pre-trained language model is performed.

Furthermore, a step S120 of acquiring the specification (spec) for the kit from the language model that received the initial proposal is performed. At this time, the acquired kit specification includes at least one of a technical specification to be used for the R&D of the kit, a resource specification for the R&D, a design specification for the kit, a regulatory approval specification that the kit must meet for approval, and a performance specification for the kit.

Furthermore, a step S130 of controlling the R&D proposal for the kit to be generated to include the acquired kit specification and the intended use information is performed.

Here, the intended use information may include at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

Meanwhile, this initial proposal may be created and acquired as a form prepared to include predetermined items is provided to the first mover who proposed the intended use information. For example, if the first mover connects to the server 100 using their own terminal 400, this form may be provided to the first mover's terminal 400. Then, the first mover may fill in the desired information, including the intended use information, in this form.

The initial proposal may be incomplete. Among these, the identity information of the first mover, whether clinical samples are possessed, and the intended use information are required information, and if at least one of these is not included, it may be difficult to create a final proposal. Accordingly, in an embodiment, if the aforementioned required item is not included in the initial proposal, the first mover may be requested to provide a description for the missing required item.

Meanwhile, as previously described in the embodiment, a language model is used. Specifically, if this language model is provided with the initial proposal in the form of a query, it operates to acquire the specification of the kit. For this purpose, as mentioned before, the language model may be implemented as a generative language model, but is not limited thereto.

Here, the language model may be provided in the server 100 or, as previously mentioned, may be provided in an external server.

Hereinafter, the language model will be described. The language model refers to a model generated based on human language. Such a language model may be acquired in various ways. The language model in the embodiment may be acquired by the transfer learning method. In the transfer learning method, as previously described, a process of pre-training a large amount of unlabeled training data (e.g., a corpus) using a semi-supervised or self-supervised learning method to obtain a pre-trained model (or base part) with a first task is performed. Thereafter, a process of fine-tuning the pre-trained model to be suitable for a second task is performed. In this fine-tuning, labeled training data is used for learning in a supervised learning method. In the embodiment, models trained via this transfer learning method include, but are not limited to, BERT (Bidirectional Encoder Representations from Transformers) or GPT (Generative Pre-trained Transformer).

Here, such a language model according to the embodiment may be ChatGPT based on GPT-3 or GPT-4. Specifically, GPT-3 is the third-generation language prediction model in the GPT-n series created by a company called OpenAI. GPT-3 consists of 175 billion parameters, making it more than twice as large as the previous version, GPT-2, introduced in May 2020. It is part of a natural language processing (NLP) system of pre-trained language.

The tasks that this GPT-3 may perform are known to include solving various language-related problems, random writing, arithmetic operations, translation, simple web coding according to a given sentence, and conversation.

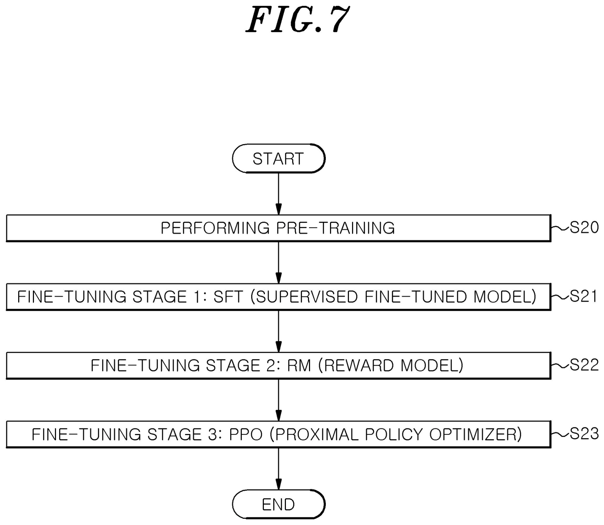

The training process of the language model is illustrated in FIG. 7. Referring to FIG. 7, the language model according to the embodiment may be pre-trained with general language (S20), or it may be pre-trained based on at least one of papers, especially biological papers, published patent applications, and published utility model applications. Alternatively, R&D documents of kits of the same or different types as the kit may be used for training.

Therefore, a more professional understanding of papers or patent literature and generation based on it becomes possible. This is because if the model is pre-trained based on papers, published patent applications, or published utility model applications using an MLM (Masked Language Model) or NSP (Next Sentence Prediction) method, this pre-trained model can learn the linguistic system of IP documents.

Furthermore, in the fine-tuning of the language model, RLHF (Reinforcement Learning from Human Feedback) may be performed. RLHF refers to the use of information judged by humans for learning in fine-tuning. For example, during the fine-tuning process in RLHF, a learning process using a dialogue set generated by a person and a learning process using a ranking selected by a person for a plurality of outputs generated by the language model may be performed. More specifically, referring to FIG. 7, an SFT (Supervised Fine-Tuned model) is generated by a dialogue set generated by a person (S21), and then a person ranks the results output by the SFT model, which is fed back to the model (RM, Reward Model) (S22), and then fine-tuning by PPO (Proximal Policy Optimization) is performed (S23). Here, the fine-tuning by PPO refers to a reinforcement learning policy algorithm, which is an algorithm that continuously adjusts the current policy based on the tasks performed by an agent and the rewards received. In PPO, the process proceeds in the order of new prompt -> PPO -> Generate output -> Calculate reward, where the Calculate reward is updated and provided again as a new prompt.

The language model may be utilized in various ways.

For example, the language model may extract, from the plurality of papers, at least one piece of paper information among the paper's authors, impact factor, and the institution to which the paper's authors belong. Then, the research and development proposal may be generated to include the at least one extracted piece of paper information.

Furthermore, the language model may be trained using big data prepared using R&D documents of kits of the same or different types as the kit. For example, a company that researches and develops kits may have various types of R&D documents stored as big data, and the language model may have access to such data and may derive or generate the aforementioned kit specification therefrom. It is a matter of course that information not disclosed in papers or the like may be acquired from this big data.

Furthermore, the language model may acquire marketability survey data for the kit. Then, the research and development proposal may be generated to include this marketability survey data.

Furthermore, the language model may acquire information necessary for the regulatory approval of the kit. The information necessary for this regulatory approval may include at least one of information on the source of clinical samples, the target country for the regulatory approval, and information on the institution where experiments for the regulatory approval are conducted. Then, the research and development proposal for the kit may be generated to further include the previously acquired information necessary for regulatory approval.

Furthermore, the language model may acquire information necessary for the production of the kit. Then, the research and development proposal for the kit may be generated to further include the acquired information necessary for the production of the kit.

Furthermore, the language model may acquire at least one of information regarding the Target Price (TP) for the sale of the kit and projected sales revenue. Then, the research and development proposal for the kit may be generated to further include at least one of the acquired information on the Target Price and the information on the projected sales revenue.

Meanwhile, required specifications may be predefined in the research and development proposal as well. For example, whether samples are available, the name of the target analyte, etc., correspond to required specifications. However, these required specifications may not be secured from papers, patents, or the aforementioned big data. Then, in the embodiment, for the reason that this required specification was not secured, it may be described in the research and development proposal for that kit that such a required specification is missing, or the research and development proposal may not be generated.

In this case, as new papers or patents are published or new technology is discovered, supplementary information for this missing item may be acquired. Then, an alarm that supplementary information has been acquired may be transmitted to the first mover or the author of the research and development proposal, or the research and development proposal may be modified or generated to include this supplementary information.

Meanwhile, in the embodiment, the author of the research and development proposal may be described as one or more persons. For example, the author may be described as at least one of the provider who provided the intended use information, the author of the paper, and the operating entity of the server for automatically generating the research and development proposal.

Furthermore, in the embodiment, the research and development proposal is subject to evaluation, and an evaluator, who is the entity to perform this evaluation, may be selected. At this time, the evaluator may be selected as at least one of: the author of the paper, the operating entity of the publication server where the paper is published, a person selected by the operating entity, the server for automatically generating the research and development proposal, and a person selected by the operating entity of the server for automatically generating the research and development proposal.

Furthermore, in the embodiment, the language model may acquire the evaluation results for the research and development proposal, summarize them, and then provide the summary.

That is, according to the embodiment, the final proposal, which is the research and development proposal for the kit, may be generated from numerous papers and patent literature using the first mover's initial proposal with the language model, and additionally, information on marketability, regulatory approval, production, and the like may also be acquired from the language model and reflected in the final proposal. Therefore, from the perspective of a company that researches and develops kits, it is possible to acquire necessary up-to-date information from vast amounts of information in the shortest time without errors, and from that, even secure a research and development proposal, without a researcher needing to examine papers or patent literature one by one to review the first mover's request. Through this, the feasibility of research and development for various types of kits may be reviewed quickly and accurately.

Meanwhile, according to the embodiment, a kit lifecycle management server and a kit lifecycle management method performed thereby may be presented. The lifecycle management server may be provided separately from the aforementioned server 100 and may be connected to the network 600 illustrated in FIG. 2.

In the lifecycle management method, a step of acquiring an initial proposal including intended use information for the kit is performed.

Furthermore, a step of providing the acquired initial proposal to a pre-trained language model is performed.

Furthermore, a step of acquiring a specification (spec) for the kit, which the language model has extracted from a plurality of papers or a plurality of papers and patent documents, is performed. At this time, the acquired kit specification may include at least one of a technical specification to be used for the R&D of the kit, a resource specification for the R&D, a design specification for the kit, and a performance specification for the kit.

Furthermore, a step of controlling a research and development proposal for the kit to be generated to include the acquired kit specification and the intended use information is performed.

Furthermore, a step of selecting an entity to research and develop the kit according to the research and development proposal is performed.

Furthermore, if the kit researched and developed by the selected entity is sold, a step of distributing revenue generated from the sales among the operating entity of the lifecycle management server, the first mover who proposed the intended use, the authors of the papers that are the source of the kit's specification, and the selected entity, according to a predetermined ratio, is performed.

Here, when the aforementioned revenue is distributed to the authors of the papers according to a certain ratio, this ratio may be dependent on the contribution of the papers.

As described above, according to the embodiment, a final proposal, which is a research and development proposal for a kit, may be generated from numerous papers and patent literature using a first mover's initial proposal with a language model, and additionally, information on marketability, regulatory approval, production, and the like may also be acquired from this language model and reflected in the final proposal. Therefore, from the perspective of a company that researches and develops kits, it is possible to acquire necessary up-to-date information from vast amounts of information in the shortest time without errors, and from that, even secure a research and development proposal, without a researcher needing to examine papers or patent literature one by one to review the first mover's request. Through this, the feasibility of research and development for various types of kits may be reviewed quickly and accurately.

Meanwhile, a review of the aforementioned initial proposal or the specification acquired for the kit may be controlled to be performed. Specifically, whether to perform a review of the initial proposal may be determined based on a result of evaluating the expertise of the first mover who proposed the initial proposal. If the expertise is above a threshold, a review may be unnecessary, but if it is below this threshold, a review may be necessary. Here, the expertise of the first mover may be evaluated in various ways. For example, it may be based on an evaluation of the first mover's background knowledge. More specifically, if the first mover publishes two or more papers on related technology annually, the expertise of that first mover up to that year may be evaluated as being above the threshold, or if five or more related patents are filed annually, the expertise of that first mover up to that year may also be evaluated as being above the threshold. Of course, the method of determining or evaluating whether the expertise is above or below the threshold is varied and is not limited to any one method.

Furthermore, a review of not only this initial proposal but also the final proposal, that is, the research and development proposal generated by the language model, may also be controlled to be performed. Here, the server 100 may perform a procedure to have this proposal delivered to a person who may perform a review, so that a review of this research and development proposal is performed. Furthermore, if a review of the research and development proposal and a review of the initial proposal are performed, the fee or cost set for the review of the research and development proposal in the server 100 may be higher than the fee or cost set for the review of the initial proposal. Therefore, if such a fee or cost is set or determined in the server 100, the server 100 may be operated to provide guidance on the criteria for determining this fee or cost.

Meanwhile, the methods according to the various embodiments described above may be implemented in the form of a computer program stored on a computer-readable recording medium programmed to perform each step of these methods, and may also be implemented in the form of a computer-readable recording medium that stores a computer program programmed to perform each step of these methods.

The above description is merely exemplary description of the technical scope of the present invention, and it will be understood by those skilled in the art that various changes and modifications can be made without departing from original characteristics of the present invention. Therefore, the embodiments disclosed in the present invention are intended to explain, not to limit, the technical scope of the present invention, and the technical scope of the present invention is not limited by the embodiments. The protection scope of the present invention should be interpreted based on the following claims and it should be appreciated that all technical scopes included within a range equivalent thereto are included in the protection scope of the present invention.

## Claims

1. A method for automatically generating a research and development proposal for an in vitro diagnostic kit, performed by a server for automatically generating the research and development proposal, the method comprising: the steps of
acquiring an initial proposal including intended use information for the kit;
providing the acquired initial proposal to a pre-trained language model;
acquiring a specification (spec) for the kit from the language model receiving the initial proposal, wherein the acquired specification for the kit includes at least one of a technical specification to be used for the research and development of the kit, a resource specification for the research and development, a design specification for the kit, a regulatory approval specification that is needed to meet for approval, and a performance specification for the kit; and
controlling to generate the research and development proposal for the kit including the acquired specification and the intended use information for the kit.

2. The method of claim 1, wherein the intended use information includes at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

3. The method of claim 1, wherein the initial proposal is created and acquired as a form prepared to allow inputting predetermined items is provided to a first mover who has proposed the intended use information, and
wherein the intended use information includes at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

4. The method of claim 3, wherein the predetermined items further include at least one of identity information of the first mover, information on whether the first mover possesses clinical samples, an amount of the possessed clinical samples, and information on a stage of the research and development of the kit in which the first mover wishes to participate, and
the method further includes, if at least one of the identity information of the first mover, the information on whether the first mover possesses clinical samples, and the intended use information as an essential information among the predetermined items is not input in the initial proposal, requesting the first mover to provide a description for the missing essential information.

5. The method of claim 1, wherein the language model is a generative model trained by RLHF (Reinforcement Learning from Human Feedback).

6. The method of claim 5, wherein the RLHF includes performing a learning process using a dialogue set generated by a human and a learning process using a ranking selected by a human for a plurality of outputs generated by the language model.

7. The method of claim 1, wherein at least one of a patent, a paper, and a research note is used for training the language model, and
wherein if the research note is used for the training, the research note includes experiment result information reported in a development completion review of the kit.

8. The method of claim 1, wherein the language model extracts, from a plurality of papers, at least one piece of paper information among authors of the plurality of papers and institutions to which the authors of the papers belong, and the research and development proposal is generated to include the at least one extracted piece of paper information.

9. The method of claim 1, wherein the technical specification of the kit includes at least one of a sampling technology, an extraction technology, a test reaction setup technology, a target signal generation technology, a signal analysis technology, and an information display technology.

10. The method of claim 9, wherein the kit is a molecular diagnostic kit, and
the technical specification of the molecular diagnostic kit includes at least one of oligo type (primer only or primer and probe), oligo structure, target-signal generation mechanism with amplification, amplification curve analysis, amplification curve viewing, and liquid handler operation information.

11. The method of claim 1, wherein the resource specification for the research and development of the kit includes at least one of information on availability of clinical samples, an amount of the possessed clinical samples if possessed, a source of the clinical samples, personnel, research funds, a research period, research equipment, and research facilities.

12. The method of claim 1, wherein the design specification of the kit includes information on at least one of an analyte panel configuration, a channel configuration, an IC, a PC, and an NC.

13. The method of claim 1, wherein the regulatory approval specification includes at least one of numerical data that is needed to satisfy to obtain approval, target analyte information for an approval process, panel configuration information according to a type of approval, information on a source of clinical samples, a target country for the approval, and information on an institution where experiments for the approval are conducted.

14. The method of claim 1, wherein the performance specification of the kit includes at least one of sensitivity, specificity, TAT (Turnaround Time), reproducibility indicating consistency of diagnostic results when a same sample is tested at different times or in different places, linearity indicating an accuracy of test results as a sample concentration increases, a limit of detection (LoD) indicating a minimum detectable sample concentration, and a range of inclusive and exclusive strains.

15. The method of claim 1, wherein research and development documents of a kit of a same or different type as the kit are used in a training process of the language model.

16. The method of claim 1, further comprising:
acquiring marketability survey data for the kit from the language model that received the initial proposal,
wherein the research and development proposal for the kit is generated to further include the acquired marketability survey data.

17. The method of claim 1, further comprising:
acquiring information necessary for production of the kit from the language model that received the initial proposal,
wherein the research and development proposal for the kit is generated to further include the acquired information necessary for the production of the kit.

18. The method of claim 1, further comprising:
acquiring at least one of information regarding a target price for sale of the kit and projected sales revenue from the language model that received the initial proposal,
wherein the research and development proposal for the kit is generated to further include at least one of the acquired information regarding the target price and the information regarding the projected sales revenue.

19. The method of claim 1, wherein one or more required specification items that is needed to be included in the research and development proposal are predefined in the server for automatically generating the research and development proposal, and
in the generating the research and development proposal for the kit, if at least a part of the one or more required specification items is missing from the acquired kit specification, the missing required specification item is indicated in the generated research and development proposal, or the research and development proposal is not generated.

20. The method of claim 19, further comprising:
when the language model acquires supplementary information for the missing required specification item from an updated or new paper and/or patent, transmitting an alarm to a first mover who proposed the intended use information and/or modifying or generating the research and development proposal to include the supplementary information for the missing required specification item.

21. The method of claim 1, wherein in the research and development proposal, an author of the research and development proposal is described as at least one of a provider who provided the intended use information and an operating entity of the server for automatically generating the research and development proposal.

22. The method of claim 1, further comprising:
selecting an evaluator to evaluate the research and development proposal.

23. The method of claim 22, wherein the evaluator is selected as at least one of an author of the paper, an operating entity of a publication server where the paper is published, a person selected by the operating entity, the server for automatically generating the research and development proposal, and a person selected by the operating entity of the server for automatically generating the research and development proposal.

24. The method of claim 1, further comprising:
acquiring an evaluation result for the research and development proposal; and
summarizing and providing the acquired evaluation result.

25. The method of claim 1, wherein the in vitro diagnostic kit includes at least one of a molecular diagnostic kit, an immunodiagnostic kit, and an antigen/antibody diagnostic kit.

26. The method of claim 1, further comprising:
controlling performance of a review on at least one of the acquired initial proposal and the specification acquired for the kit.

27. The method of claim 26, further comprising:
evaluating an expertise of a first mover who has proposed the initial proposal by using a background knowledge of the first mover,
wherein whether to perform the review is determined based on a result of the evaluation of the background knowledge of the first mover.

28. The method of claim 1, further comprising:
controlling performance of a review on the generated research and development proposal.

29. A computer-readable recording medium storing a computer program programmed to perform each step included in the method of claim 1.

30. A computer program stored on a computer-readable recording medium, the computer program being programmed to perform each step included in the method of claim 1.

31. A server for automatically generating a research and development proposal, comprising:
a memory storing at least one instruction; and
a processor,
wherein when the at least one instruction is executed by the processor,
an initial proposal including intended use information for an in vitro diagnostic kit is acquired,
the acquired initial proposal is provided to a pre-trained language model,
a specification (spec) for the kit is acquired from the language model that received the initial proposal, wherein the acquired specification for the kit includes at least one of a technical specification to be used for the research and development of the kit, a resource specification for the research and development, a design specification for the kit, a regulatory approval specification that is needed to meet for approval, and a performance specification for the kit, and
a research and development proposal for the kit is generated to include the acquired specification for the kit and the intended use information.

32. The server for automatically generating the research and development proposal of claim 31, wherein the intended use information includes at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

33. The server for automatically generating the research and development proposal of claim 31, wherein the initial proposal is created and acquired as a form prepared to include predetermined items is provided to a first mover who proposed the intended use information, and
wherein the intended use information includes at least one of a diagnostic test purpose, a diagnostic test method, a specimen type, target analyte information to be detected or analyzed, pathogen information, information on contaminants, information about a pathogenic gene or SNP (Single Nucleotide Polymorphism), a disease name, an infectious disease name, and symptom information.

34. The server for automatically generating the research and development proposal of claim 33, wherein the predetermined items further include at least one of identity information of the first mover, whether clinical samples are possessed, an amount of the possessed clinical samples, and information on a stage of the research and development of the kit in which the first mover wishes to participate, and
wherein when the at least one instruction is executed by the processor,
if at least one of the identity information of the first mover, the information on whether clinical samples are possessed, and the intended use information, which correspond to required information among the predetermined items, is not included in the initial proposal, the first mover is requested to provide a description for the corresponding item.

35. The server for automatically generating the research and development proposal of claim 31, wherein the language model is a generative model trained by RLHF (Reinforcement Learning from Human Feedback).

36. The server for automatically generating the research and development proposal of claim 35, wherein the RLHF includes performing a learning process using a dialogue set generated by a human and a learning process using a ranking selected by a human for a plurality of outputs generated by the language model.

37. The server for automatically generating the research and development proposal of claim 31, wherein at least one of a patent, a paper, and a research note is used for training the language model, and
wherein if the research note is used for the training, the research note includes experiment result information reported in a development completion review of the kit.

38. The server for automatically generating the research and development proposal of claim 31, wherein the language model extracts, from a plurality of papers, at least one piece of paper information among authors of the plurality of papers and institutions to which the authors of the papers belong, and
the research and development proposal is generated to include the at least one extracted piece of paper information.

39. The server for automatically generating the research and development proposal of claim 31, wherein the technical specification of the kit includes at least one of a sampling technology, an extraction technology, a test reaction setup technology, a target signal generation technology, a signal analysis technology, and an information display technology.

40. The server for automatically generating the research and development proposal of claim 39, wherein the kit is a molecular diagnostic kit, and
the technical specification of the molecular diagnostic kit includes at least one of oligo type (primer only or primer and probe), oligo structure, target-signal generation mechanism with amplification, amplification curve analysis, amplification curve viewing, and liquid handler operation information.

41. The server for automatically generating the research and development proposal of claim 31, wherein the resource specification for the research and development of the kit includes at least one of information on availability of clinical samples, an amount of the possessed clinical samples if possessed, a source of the clinical samples, personnel, research funds, a research period, research equipment, and research facilities.

42. The server for automatically generating the research and development proposal of claim 31, wherein the design specification of the kit includes information on at least one of an analyte panel configuration, a channel configuration, an IC, a PC, and an NC.

43. The server for automatically generating the research and development proposal of claim 31, wherein the regulatory approval specification includes at least one of numerical data that the kit must satisfy to obtain approval, target analyte information for an approval process, panel configuration information according to a type of approval, information on a source of clinical samples, a target country for the approval, and information on an institution where experiments for the approval are conducted.

44. The server for automatically generating the research and development proposal of claim 31, wherein the performance specification of the kit includes at least one of sensitivity, specificity, TAT (Turnaround Time), reproducibility indicating consistency of diagnostic results when a same sample is tested at different times or in different places, linearity indicating an accuracy of test results as a sample concentration increases, a limit of detection (LoD, Limit of Detection) indicating a minimum detectable sample concentration, and a range of inclusive and exclusive strains.

45. The server for automatically generating the research and development proposal of claim 31, wherein research and development documents of a kit of a same or different type as the kit are used in a training process of the language model.

46. The server for automatically generating the research and development proposal of claim 31, wherein when the at least one instruction is executed by the processor, marketability survey data for the kit is acquired from the language model that received the initial proposal, and
the research and development proposal for the kit is generated to further include the acquired marketability survey data.

47. The server for automatically generating the research and development proposal of claim 31, wherein when the at least one instruction is executed by the processor, information necessary for production of the kit is acquired from the language model that received the initial proposal, and
the research and development proposal for the kit is generated to further include the acquired information necessary for the production of the kit.

48. The server for automatically generating the research and development proposal of claim 31, wherein when the at least one instruction is executed by the processor, at least one of information regarding a target price for sale of the kit and projected sales revenue is acquired from the language model that received the initial proposal, and
the research and development proposal for the kit is generated to further include at least one of the acquired information regarding the target price and the information regarding the projected sales revenue.

49. The server for automatically generating the research and development proposal of claim 31, wherein one or more required specification items that is needed to be included in the research and development proposal are predefined and stored in the memory, and
the research and development proposal is generated with the missing required specification item indicated, or is not generated, when at least a part of the one or more required specification items is missing from the acquired kit specification.

50. The server for automatically generating the research and development proposal of claim 49, wherein when the language model acquires supplementary information for the missing required specification item from an updated or new paper and/or patent, an alarm is transmitted to a first mover who proposed the intended use information and/or the research and development proposal is modified or generated to include the supplementary information for the missing required specification item.

51. The server for automatically generating the research and development proposal of claim 31, wherein in the research and development proposal, an author of the research and development proposal is described as at least one of a provider who provided the intended use information and an operating entity of the server for automatically generating the research and development proposal.

52. The server for automatically generating the research and development proposal of claim 31, wherein when the at least one instruction is executed by the processor, an evaluator to evaluate the research and development proposal is selected.

53. The server for automatically generating the research and development proposal of claim 52, wherein the evaluator is selected as at least one of an author of the paper, an operating entity of a publication server where the paper is published, a person selected by the operating entity, an operating entity of the server for automatically generating the research and development proposal, and a person selected by the operating entity of the server for automatically generating the research and development proposal.

54. The server for automatically generating the research and development proposal of claim 31, wherein when the at least one instruction is executed by the processor, an evaluation result for the research and development proposal is acquired, and the acquired evaluation result is summarized and provided.

55. The server for automatically generating the research and development proposal of claim 31, wherein the in vitro diagnostic kit includes at least one of a molecular diagnostic kit, an immunodiagnostic kit, and an antigen/antibody diagnostic kit.

56. The server for automatically generating the research and development proposal of claim 31, wherein when the at least one instruction is executed by the processor,
a review is controlled to be performed on at least one of the acquired initial proposal and the specification acquired for the kit.

57. The server for automatically generating the research and development proposal of claim 56, wherein when the at least one instruction is executed by the processor,
an expertise of a first mover who proposed the initial proposal is evaluated by using a background knowledge of the first mover, and
whether to perform the review is determined based on a result of the evaluation of the background knowledge of the first mover.

58. The server for automatically generating the research and development proposal of claim 31, wherein when the at least one instruction is executed by the processor, a review of the generated research and development proposal is controlled to be performed.
